# EUROPÄISCHE PATENTSCHRIFT

(11) **EP 2 357 246 B1**
(45) Veröffentlichungstag und Bekanntmachung des Hinweises auf die Patenterteilung: **22.08.2012**
(21) Anmeldenummer: 11154915.0
(22) Anmeldetag: 17.02.2011
(51) Int. Cl.: C12P 7/56, C12P 5/02, C12M 1/107

(54) **Integriertes Verfahren zur Herstellung von Ammoniumlactat**
Integrated process for producing ammonium lactate
Procédé intégré de production de lactate d'ammonium

(30) Priorität: 17.02.2010 DE 102010002065
(43) Veröffentlichungstag der Anmeldung: 17.08.2011
(73) Patentinhaber: Deutsches Institut für Lebensmitteltechnik e.V., 49610 Quakenbrück (DE)
(72) Erfinder: Hertel, Christian, Dr., 49610 Quakenbrück (DE); Heinz, Volker PD Dr., 49610 Quakenbrück (DE)
(74) Vertreter: Taruttis, Stefan Georg

(56) Entgegenhaltungen:
- EP-A1- 2 239 333
- WO-A2-01/81610
- WO-A2-2009/090476
- DE-A1- 2 731 877

## Beschreibung

Die vorliegende Erfindung betrifft Verfahren und Vorrichtung zur integrierten fermentativen Herstellung von Ammoniumlactat, bei dem alternativ oder optional zusätzlich Calcium-Ammoniumlactat, NH₃ als Gas oder in wässriger Lösung und/oder Zusammensetzungen mit probiotischen Bakterienkulturen entstehen.

Die Erfindung verwendet die fermentative Herstellung von Milchsäure bzw. Lactat aus einem Substrat, das Protein und/oder Saccharid einschließlich Stärke enthält, insbesondere Molke, die vorzugsweise Süßmolke, Sauermolke und/oder deproteinierte Molke, wahlweise aus Pulver hergestellt ist, wobei das Substrat fermentativ mittels heterofermentativer und/oder homofermentativer Mikroorganismen zu Lactat umgesetzt wird. Mikroorganismen können unter Pilzen, insbesondere submers kultivierbaren Pilzen, z.B. Rhizopus sp., z.B. Rhizopus oryzae, Actinomucor sp., Amylomyces sp., z.B. Amylomyces rouxii, und/oder homo- oder heterofermentativen Milchsäurebakterien, z.B. Lactobacillen, Leuconostoc, Streptococcus, die optional amylolytisch sind, ausgewählt werden. Während dieser Milchsäurefermentation wird Ammoniak, gasförmig oder in wässriger Lösung, zugesetzt und/oder im Anschluss an die satzweise, semi-kontinuierliche oder kontinuierliche Milchsäurefermentation wird Fermenterbrühe mit Ammoniak versetzt, der gasförmig oder in wässriger Lösung vorliegt. Auf diese Weise in der Fermenterbrühe hergestelltes Ammoniumlactat kann in herkömmlicher Weise isoliert werden, vorzugsweise durch Dialyseverfahren, insbesondere mittels Elektrodialyse, oder durch Ausfällung, beispielsweise als Calcium-Ammoniumlactat mittels Zugabe eines Calciumsalzes.

### Stand der Technik

Die DE 2731877 beschreibt die fermentative Herstellung von Ammoniumlactat aus Molke, wobei der Fermenterbrühe Ammoniak umgesetzt wird.

Die WO 2009/090476 A2 beschreibt ein integriertes Verfahren zur mikrobiellen Umsetzung organischen Abfalls zur Erzeugung von Biogas und eines Rückstands, von dem eine wässrige Fraktion abgetrennt wird und einem zweiten Modul neben weiteren Ausgangsstoffen zugeführt wird.

Die WO 01/81610 A2 beschreibt die Herstellung von Lactat durch mikrobielle Umsetzung einer wässrigen Fermenterbrühe, in der hydrolysiertes Getreidemehl als Substrat enthalten ist. Zurückbleibende Biomasse kann zu Biogas umgesetzt werden.

Die EP 2239333 A1, die nur gemäß Artikel 54 (3) EPÜ relevant ist, beschreibt die fermentative Umsetzung wässriger organischer Zusammensetzungen, bevorzugt zu organischen Säuren, einschließlich Milchsäure. Die organischen Säuren können durch Ionenaustausch abgetrennt werden und beispielsweise mit Ammoniak eluiert werden, das aus der Biogasfermentation von lignocellulosischem Material mittels Calciumhydroxid abgetrennt wurde.

Die EP 97911585 (entsprechend Publication EP0932542 B1) beschreibt ein besonderes Verfahren zum Isolieren von Lactat aus Lösung.

Die EP 0657542 A1 beschreibt die Abtrennung von Lactat aus Fermenterbrühe mittels Elektrodialyse, wobei der pH durch Zugabe von Ammoniak eingestellt wird.

Die US 4,333,956 und US 4,420,636 beschreiben die Herstellung von Lecksteinen für Rinder durch Zugabe von Calciumsalz zu Ammoniumlactat, das fermentativ aus Molke hergestellt wurde. Bei Temperaturen von unterhalb etwa 30 °C wurde die Verfestigung des Calcium-Ammoniumlactats beobachtet.

Die WO 01/92555 beschreibt die Fermentation von Molke zu Lactat unter Zusatz von gasförmigem Ammoniak und die anschließende Aufarbeitung mittels Ultrafiltration zur Abtrennung freier Milchsäure, die mit weiteren Filtrations- und Elektrodialyseschritten aufgereinigt werden kann.

Die WO 2007/013259 und die US 5,453,365 beschreiben die Ausfällung von Ammoniumlactat aus Fermenterbrühe von Molke und die anschließende Veresterung zum Milchsäureester.

John et al. (Biotechnology Advances, 145-152 (2009)) beschreiben die direkte Verzuckerung und Milchsäuregärung auf Basis von Stärke mittels amylolytischer Milchsäurebakterien und Pilzen.

### Aufgabe der Erfindung

Die Aufgabe der Erfindung liegt in der Bereitstellung eines alternativen Verfahrens zur Produktion von Ammoniumlactat, das wirtschaftlich günstig sein soll.

### Allgemeine Beschreibung der Erfindung

Die Erfindung löst die Aufgabe mit den Merkmalen der Ansprüche und insbesondere mit einem Verfahren zur fermentativen Herstellung von Ammoniumlactat aus einem ersten Substrat, das ein Saccharid enthält, optional Stärke und/oder Protein, mittels Mikroorganismen in einer ersten Fermenterbrühe, auch Milchsäurefermentation genannt, die stofflich und energetisch mit einer Fermentation zur Produktion von Biogas aus einer zweiten Fermenterbrühe gekoppelt ist. Weiterhin betrifft die Erfindung eine Vorrichtung und deren Verwendung für das Verfahren.

Das erste Substrat in wässriger Lösung, das der ersten Fermenterbrühe zur Lactatfermentation zugesetzt wird, enthält ein Saccharid und kann einen hohen Anteil Protein aufweisen oder daraus bestehen und optional frei von Stärke sein. Das erste Substrat ist bevorzugt ein sog. Nebenstrom oder Nebenprodukt der Lebensmittelherstellung, wie z.B. Molke, Melasse, Kartoffelpulpe, Kartoffelfruchtwasser, Erbsenpulpe und/oder Erbsenfruchtwasser, das jeweils stärkefrei ist und z.B. durch Abtrennung der Stärke aus Kartoffeln bzw. Erbsen gewonnen ist, oder eine Mischung daraus.

Eine zweite Fermentation zur Erzeugung von Biogas, auch Biogasfermentation genannt, weist die Vergärung von stickstoffhaltigen Substraten in Mischung mit Wasser auf, die insbesondere proteinreiche organische Reststoffe, z.B. Schlachtabfälle, Bioabfälle, Lebensmittelrückstände, und/oder harnstoff und/oder harnsäurehaltige tierische Exkremente, z.B. landwirtschaftliche Gülle, enthalten oder daraus bestehen. Die stoffliche Kopplung der Milchsäurefermentation mit der Biogasfermentation hat den besonderen Vorteil, dass die für die Milchsäurefermentation erforderliche pH-abhängig gesteuerte Zugabe von NH₃ bzw. Ammonium zur ersten Fermenterbrühe für die Pufferung in Form von gasförmigem NH₃ oder Ammoniak bzw. NH₃ in wässriger Lösung als Ammonium erfolgt, das aus der fermentativen Biogasherstellung stammt. Auf diese Weise wird Ammoniak (NH₃), gasförmig bzw. in wässriger Lösung, das in der Biogasfermentation Hemmstoff ist, aus der zweiten Fermenterbrühe der Biogasfermentation abgetrennt, insbesondere ausgestrippt, und zumindest anteilig der Milchsäurefermentation zugegeben, insbesondere dieser in Abhängigkeit vom pH-Wert der zweiten Fermenterbrühe gesteuert zugegeben. Dabei hat sich herausgestellt, dass Verunreinigungen, die im Abgas der Biogasfermentation oder in der aus der Fermenterbrühe der Biogasfermentation ausgestrippten NH₃ -haltigen Phase enthalten sind, bei unmittelbarer Einleitung in die Milchsäurefermentation nicht zu einer wesentlichen Beeinträchtigung der Produktion von Ammoniumlactat führen. Optional wird die ausgestrippte Phase vor Einleitung von NH₃ in die erste Fermenterbrühe, wobei das NH₃ insbesondere gasförmig vorliegt oder in wässriger Lösung, die durch Kontaktieren des gasförmigen NH₃ mit Wasser erzeugt ist, einer Gasreinigung unterzogen, z.B. durch Waschen der ausgestrippten Gasphase mit einem organischen Lösungsmittel, z.B. mit Methanol, oder mittels eines Membrantrennverfahrens oder Waschen mit Wasser. Die Unempfindlichkeit der Milchsäurefermentation gegenüber Begleitstoffen, die in NH₃ -haltiger Abluft oder in NH₃ - haltigem Strippgas der Biogasfermentation enthalten sind, ist insofern überraschend, als bislang in Milchsäurefermentationen für die Herstellung von Futter- oder Lebensmittelzusätzen NH₃ im Wesentlichen in reiner Form zugeführt wurde. Daher bietet die erfindungsgemäße Verwendung von NH₃ aus Abgas und/oder Strippgas der Biogasfermentation den Vorteil, dass NH₃ optional ohne besondere Aufreinigung oder nach einer Gasreinigung der aus der zweiten Fermenterbrühe ausgestrippten NH₃ -haltige Phase als Puffersubstanz in der Herstellung von Ammoniumlactat eingesetzt wird. Optional kann NH₃ - haltige Abluft der Biogasfermentation der ausgestrippten NH₃ -haltigen Phase zugesetzt sein oder diese bilden.

In einer bevorzugten Ausführungsform, wird gasförmiges NH₃, z.B. in Mischung mit nicht wasserlöslichem Gas, z.B. als ein aus der zweiten Fermenterbrühe ausgestripptes Gas oder in Form von Abluft aus der Biogasfermentation bzw. aus dem zweiten Gärungsbehälter, in die erste Fermenterbrühe eingeleitet wobei das NH₃-haltige Gas optional eine Einrichtung zur Gasreinigung durchlaufen hat. Dabei wird das aus der ersten Fermenterbrühe, d.h. der Milchsäurefermentation, austretende Abgas als Strippmedium zum Ausstrippen von NH₃ aus der zweiten Fermenterbrühe eingesetzt, in der die Biogasfermentation abläuft. Bei dieser Rückführung der Abluft aus dem Gärungsbehälter, in dem die erste Fermenterbrühe der Milchsäurefermentation enthalten ist, als Strippmedium in eine Strippeinrichtung, in der NH₃ aus zweiten Fermenterbrühe der Biogasfermentation gestrippt wird, wird bevorzugt die Menge insgesamt austretender Abluft vermindert.

Erfindungsgemäß ist die Milchsäurefermentation stofflich auch dadurch mit der Biogasfermentation gekoppelt, dass die nach Abtrennung von Ammoniumlactat aus der ersten Fermenterbrühe der Milchsäurefermentation zurückbleibende Fraktion zumindest anteilig, vorzugsweise vollständig, in den Gärungsbehälter der Biogasfermentation eingeleitet wird. Auf diese Weise wird Biomasse, die durch die Milchsäurefermentation erzeugt wird und/oder nach Abtrennung von Lactat zurückbleibt, mit dem erfindungsgemäßen Verfahren zumindest anteilig zu Biogas umgesetzt. Die organischen Verbindungen, die nach Abtrennung von Ammoniumlactat in der Fraktion der ersten Fermenterbrühe enthalten sind, die in die zweite Fermenterbrühe geleitet wird, werden in der zweiten Fermenterbrühe umgesetzt, z.B. zu Biogas. Daher können z.B. Anteile des ersten Substrats, die in der Milchsäurefermentation nicht zu Milchsäure umgesetzt werden bzw. nicht verstoffwechselt werden, in der zweiten Fermenterbrühe durch die Biogasfermentation umgesetzt werden. Daher ermöglicht das Verfahren die Umsetzung der Anteile des ersten Substrats und der Mikroorganismen, die in der ersten Fermenterbrühe nach Abtrennung von Ammoniumlactat enthalten sind, zu Biogas, während für die Milchsäurefermentation ein erstes Substrat mit geringem Saccharidanteil bzw. hohem Proteinanteil verwendet werden kann. Entsprechend weist das erfindungsgemäße Verfahren den Schritt auf, die nach Abtrennung von Ammoniumlactat aus der ersten Fermenterbrühe der Milchsäurefermentation erhaltene Fraktion zumindest anteilig, vorzugsweise vollständig, in den Gärungsbehälter der Biogasfermentation zu leiten.

Für die Zwecke der Erfindung umfasst der Begriff Fermenterbrühe wässrige Zusammensetzungen, die abhängig vom Gehalt an Trockensubstanz flüssig bis pastös sind, und vorzugsweise pumpfähig. Als Biogas wird insbesondere ein Gasgemisch mit einem Gehalt an Methan, H₂, N₂, CO₂, NH₃ und/oder H₂S bezeichnet, das insbesondere bei der anaeroben mikrobiellen Vergärung von stickstofflialtigem Substrat, z.B. proteinhaltigen Medien erzeugt wird. Die zweite Fermenterbrühe kann einen hohen Feststoffanteil aufweisen, z.B. flüssig bis pastös sein, und ist vorzugsweise pumpfähig.

Das Medium bzw. erste Substrat für die Milchsäurefermentation ist z.B. ausgewählt aus der Gruppe, die stärkehaltige und saccharidhaltige Nebenprodukte der Lebensmittelindustrie, insbesondere Melasse, Molke, proteinhaltige Abwässer, insbesondere Kartoffelfruchtwasser, Kartoffelpulpe, Erbsenpulpe und/oder Erbsenfruchtwasser und Mischungen dieser, optional stärkefrei, enthält oder daraus besteht.

Das Medium für die Biogasfermentation ist z.B. ausgewählt aus der Gruppe, die stickstoffhaltige Substrate wie proteinhaltige, harnstoff und harnsäurehaltige Verbindungen, insbesondere tierische Exkremente, z.B. von Schweinen oder Geflügel, proteinhaltige Abwässer, insbesondere Schlachtabfälle enthält oder daraus besteht. Da das erfindungsgemäße Verfahren durch die stoffliche Kopplung der Biogasfermentation mit der Milchsäurefermentation die stoffliche Bindung von NH₃ in dem erzeugten Produkt Ammoniumlactat ergibt, sind stickstoffhaltige Substrate für die Biogasfermentation geeignet, wie z.B. proteinhaltiges Medium und tierische Exkremente. Dies ist ein besonderer Vorteil des Verfahrens, da das aus solchen stickstoffhaltigen Substraten entstehende NH₃ in der bislang praktizierten Biogasfermentation zur Hemmung der Bildung von Biogas führt, bzw. NH₃ separat abgetrennt wird.

Zusätzlich zu der stofflichen Kopplung der Milchsäurefermentation dadurch, dass dieser ersten Fermenterbrühe NH₃ aus der Biogasfermentation zugesetzt wird und die Fraktion der ersten Fermenterbrühe der Milchsäurefermentation, die durch Abscheidung von Ammoniumlactat erhalten wird, der Biogasfermentation zugeführt wird, erfolgt eine energetische Kopplung der Biogasfermentation mit der Milchsäurefermentation. Diese energetische Kopplung nutzt die bei der Biogasfermentation anfallende Abwärme zur Temperierung der Fermenterbrühe der Milchsäurefermentation. Die Abwärme der Biogasfermentation kann unmittelbar aus deren zweiter Fermenterbrühe abgeführt werden und/oder Abwärme sein, die bei der energetischen Verwertung von Biogas anfällt, z.B. bei dessen Verbrennung in einer Heizung oder einem mit Biogas betriebenen Motor. Diese energetische Kopplung ist vorteilhaft, weil einerseits die Biogasfermentation überschüssige Abwärme erzeugt, die abzuführen ist, und andererseits die Milchsäurefermentation Temperaturen von mindestens 30 °C erfordert, vorzugsweise mindestens 35 bis 45 °C je nach Produktionsstamm der Mikroorganismen, insbesondere von Lactobacillen, die durch Erwärmung der Fermenterbrühe erreicht werden müssen, da die Milchsäurefermentation selbst nicht hinreichend exotherm abläuft. Die energetische Kopplung der Milchsäurefermentation (auch Molkefermentation genannt) mit einer Biogasfermentation ist insbesondere dann vorteilhaft, wenn das Verfahren bei niedrigeren Umgebungstemperaturen verwendet wird, und/oder der die erste Fermenterbrühe der Milchsäurefermentation enthaltene Behälter keine weitere eigene Heizung hat, bzw. bei Umgebungstemperaturen aufgestellt ist.

Generell enthält das Verfahren zur Herstellung von Ammoniumlactat die mikrobielle Umsetzung eines Substrats in einer wässrigen Fermenterbrühe zu einer Lactat enthaltenden ersten Fermenterbrühe, mit dem Schritt der Zugabe von NH₃, die insbesondere in Abhängigkeit vom pH-Wert geregelt ist, zu dieser ersten Fermenterbrühe und Abtrennung von Ammoniumlactat von der ersten Fermenterbrühe, sowie die methanogene mikrobielle Umsetzung von Biomasse, die stickstoffhaltige Verbindungen, insbesondere Protein und/oder tierische Exkremente enthält oder daraus in Wasser besteht, in einer zweiten Fermenterbrühe zu einem Biogas mit einem Gehalt an Methan, wobei das der ersten, Lactat enthaltenden Fermenterbrühe zugegebene NH₃ durch die methanogene mikrobielle Umsetzung in der zweiten Fermenterbrühe und Abtrennen von NH₃ aus der zweiten Fermenterbrühe erzeugt wird, wobei aus der zweiten Fermenterbrühe Wärme abgeführt wird und diese Wärme der ersten Fermenterbrühe zugeführt wird und wobei eine nach Abtrennen von Ammoniumlactat aus der ersten Fermenterbrühe erhaltene Fraktion der zweiten Fermenterbrühe zugeführt wird. Bevorzugt erfolgt das Abtrennen von NH₃ aus der zweiten Fermenterbrühe durch Ausstrippen einer wässrigen Fraktion der zweiten Fermenterbrühe, insbesondere mit dem Schritt des Einstellens eines neutralen oder alkalischen pH-Werts in einem wässrigen Biomasserückstand der zweiten Fermenterbrühe und Ausstrippen dieses wässrigen Biomasserückstands. Besonders bevorzugt wird das ausgestrippte NH₃-haltige Gas in die erste Fermenterbrühe geleitet und besonders bevorzugt wird das Abgas aus der ersten Fermenterbrühe als Strippmedium zum Ausstrippen der wässrigen Fraktion der zweiten Fermenterbrühe verwendet.

Die zur Durchführung des Verfahrens zur Herstellung von Ammoniumlactat verwendete Vorrichtung weist einen ersten Gärungsbehälter auf, der zur Aufnahme einer wässrigen Lösung eines ersten Substrats und einer ersten Fermenterbrühe eingerichtet ist und eine erste Zuleitung für NH₃, gasförmig oder in wässriger Lösung, und eine Entnahmeleitung zur Entnahme von Lactat enthaltender erster Fermenterbrühe aufweist, wobei die erste Zuleitung mit einer mit einem zweiten Gärungsbehälter verbundenen Auslassleitung für NH₃ verbunden ist, wobei der zweite Gärungsbehälter für die methanogene mikrobielle Umsetzung von stickstoffhaltigem zweiten Substrat, insbesondere Biomasse, zu einem Biogas mit einem Gehalt an Methan und NH₃-haltiger zweiter Biogasfermenterbrühe eingerichtet ist. Vorzugsweise ist zwischen dem zweiten Gärungsbehälter und der ersten Zuleitung eine Trenneinrichtung, z.B. ein Separator, mit einem Auslass für eine Flüssigphase und einem Auslass für eine Festphase angeordnet, an dessen Auslass für eine Flüssigphase eine mit einer Zuleitung für Strippmedium versehene Strippeinrichtung zum Ausstrippen eines NH₃-haltigen Gases angeschlossen ist, deren Auslassleitung mit der ersten Zuleitung verbindbar ist. Da das Strippmedium erfindungsgemäß bevorzugt Abluft der ersten Fermenterbrühe bzw. aus dem ersten Gärungsbehälter ist, ist die Zuleitung für Strippmedium vorzugsweise mit einer Abluftleitung bzw. Abgasleitung des ersten Gärungsbehälters verbunden. Optional ist in der Auslassleitung des zweiten Gärungsbehälters bzw. in der Zuleitung für gasförmiges NH₃ des ersten Gärungsbehälters ein Pufferbehälter zur Aufnahme von NH₃ angeordnet, das gasförmig oder in wässriger Lösung vorliegt. Der erste Gärungsbehälter kann an seiner Entnahmeleitung zur Entnahme von wässriger Lösung mit einem Gehalt an Ammoniumlactat mit einer Elektrodialyseeinrichtung verbunden sein, welche zur Abtrennung von Ammoniumlactat aus der wässrigen Lösung eingerichtet ist. Diese Entnahmeleitung kann in einen Mischbehälter führen, welcher mit einer Quelle für ein Calciumsalz verbunden ist, wobei der Mischbehälter vorzugsweise ein Absetzbehälter ist, der an seinem unteren Ende eine Austragseinrichtung zum Austragen ausgefällten Calcium-Ammoniumlactats aufweist. Diese Auslassleitung für NH₃ kann mit einer Gasreinigungseinrichtung verbunden sein, deren Auslassöffnung für die Gasphase mit der Zuleitung für NH₃ des ersten Gärungsbehälters verbunden ist.

Optional weist das Verfahren den Schritt auf, das aus der ersten Fermenterbrühe abgetrennte Ammoniumlactat mit zugesetztem Alkohol zu verestern, z.B. mit Butanol zu Butyllactatester, vorzugsweise mit Abtrennung des Alkohollactatesters und dessen Hydrolyse. Alternativ kann das Verfahren den Schritt enthalten, Ammoniumlactat zu Lactat oder Dilactid umzusetzen, z.B. durch Ansäuern einer wässrigen Lösung des Ammoniumlactats und Extraktion des Lactats, insbesondere mit einem Alkylamin und destillativer Trennung des Alkylamins von Lactat oder thermischer Zersetzung von Alkylamin und Lactat zu Oligolactid.

### Genaue Beschreibung der Erfindung

Die Erfindung wird nun genauer anhand von Beispielen mit Bezug auf die Figur beschrieben, die einen schematischen Ablauf des erfindungsgemäßen Verfahrens und ein Schema der dabei verwendbaren Vorrichtung zeigt, mit der das erfindungsgemäße Verfahren durchführbar ist. Das Verfahren zur Herstellung von Ammoniumlactat kann auch Verwendung finden zur Herstellung von freier Milchsäure, bzw. zur Herstellung von probiotischen Präparaten, die Lactobacillen enthalten, wahlweise mit einem Gehalt an Ammoniumlactat oder Lactat, sowie zur Herstellung von Calcium-Ammoniumlactat. Weiterhin eignet sich das erfindungsgemäße Verfahren zur gleichzeitigen Herstellung von Ammoniak, gasförmig oder als wässrige Lösung, als Nebenprodukt. Entsprechend betrifft die Erfindung neben der Herstellung von Ammoniumlactat optional zusätzlich die Herstellung von probiotischen Präparaten mit einem Gehalt an Milchsäurebakterien und/oder die Herstellung von Calcium-Ammoniumlactat und/oder von freier Milchsäure, insbesondere aus Ammoniumlactat, wahlweise in Kombination mit der Herstellung von Ammoniak, gasförmig oder in wässriger Lösung.

Wie in der schematischen Übersicht der Figur gezeigt ist, wird erfindungsgemäß die ein erstes Substrat, z.B. Lactose, enthaltende wässrige Zusammensetzung, auch als wässrige Fermenterbrühe 1 bezeichnet, mit Milchsäurebakterien fermentativ zu einer Lactat enthaltenden ersten Fermenterbrühe 2 umgesetzt. Bevorzugte Milchsäurebakterien sind Lactobacillen, insbesondere Lactobacillus amylovorus, Lactobacillus amylophilus, Lactobacillus coryneformis, Lactobacillus paracasei, Lactobacillus helveticus, Lactobacillus delbrueckii, Lactobacillus acidophilus, Lactobacillus casei, Lactobacillus farciminis, Lactobacillus plantarum und/oder Lactobacillus rhamnosus, wahlweise in Kombination mit Lactat erzeugenden oder Lactat toleranten Hefen oder submers kultivierbaren Pilzen, z.B. Rhizopus sp., Rhizopus arrhizus, Rhizopus oryzae und Actinomucor sp. Das erste Substrat für die Milchsäurefermentation kann eine wässrige, Stärke enthaltende oder stärkefreie, ein Saccharid, insbesondere Lactose enthaltende Zusammensetzung sein, die insbesondere Kartoffelpulpe, Erbsenpulpe, stärkefreies Kartoffelfruchtwasser, stärkefreies Erbsenfruchtwasser, Melasse, Molke, wahlweise deproteinierte Molke, Süßmolke, Sauermolke und Molkereiabwässer, die wahlweise pulverförmig und in Wasser verteilt sind, aufweist oder daraus besteht.

Bevorzugte Temperaturen für die Lactatfermentation sind im Bereich von mindestens 30 bis mindestens 45 °C, bevorzugter mindestens 35 °C. Zur Einstellung bzw. zur Regulierung des pH-Werts wird die Lactose enthaltende Fermenterbrühe 1 vor und/oder während der Fermentation und/oder die fermentativ erhaltene Lactat enthaltende Fermenterbrühe 2 mit NH₃ versetzt, wahlweise gasförmig oder in wässriger Lösung. Generell ist es für die Erfindung bevorzugt, NH₃ gasförmig zu der Lactose und/oder Lactat enthaltenen Fermenterbrühe 1, 2 zuzusetzen, da dabei das Flüssigkeitsvolumen im wesentlichen nicht vergrößert wird und eine ungewollte Verdünnung vermieden wird, wobei sich durch die Rückfiihrung der Abluft aus dem ersten Gärungsbehälter der Milchsäurefermentation in die Strippeinrichtung, in der aus der zweiten Fermenterbrühe der Biogasfermentation NH₃ ausgestrippt wird, der Vorteil der Verringerung oder Vermeidung von Abluft aus dem Verfahren bzw. aus der Vorrichtung ergibt.

Aus der das erste Substrat für die Milchsäurefermentation, insbesondere Lactat, enthaltenen Fermenterbrühe 2 wird das darin enthaltene Ammoniumlactat 3 abgetrennt, beispielsweise durch Elektrodialyse, Ultrafiltration, chromatographisch und/oder durch Ausfällung. Zur Ausfällung kann die Lactat enthaltende erste Fermenterbrühe 2 mit einem Calciumsalz versetzt werden, beispielsweise Calciumchlorid, -sulfat oder -phosphat, und Ammoniumlactat 3 kann als Calcium-Ammoniumlactat 5 ausgefällt und abgetrennt werden.

Alternativ oder zusätzlich zur Abtrennung von Ammoniumlactat 3 kann aus der Lactat enthaltenen Fermenterbrühe 2 freie Milchsäure 6 abgetrennt werden, beispielsweise durch Ultrafiltration, Elektrodialyse oder chromatographische Verfahren. Die Fraktion der Lactat enthaltenden ersten Fermenterbrühe 2, von der zumindest ein Teil des Ammoniumlactats abgetrennt ist, wird erfindungsgemäß, vorzugsweise unmittelbar oder nach Lagerung in einem Pufferbehälter 38, in den zweiten Gärungsbehälter 31 geleitet. Mikroorganismen 4, insbesondere Milchsäurebakterien, können aus einem Teil der Fraktion, die durch Abtrennung von Ammoniumlactat aus der Lactat enthaltenen ersten Fermenterbrühe 2 erhalten wird, abgetrennt werden, um beispielsweise durch Eindicken oder Trocknen ein probiotisches Präparat 4a herzustellen, das insbesondere Verwendung als Tierfutter finden kann.

Die erfindungsgemäße Kopplung der mikrobiellen Umsetzung von Lactose zu Lactat mit einem Verfahren zur Erzeugung von Biogas aus Biomasse erfolgt dadurch, dass NH₃, das der Lactose oder Lactat enthaltenden ersten Fermenterbrühe 1, 2 zugesetzt wird, aus dem wässrigen Biomasserückstand 12 abgetrennt wird, der nach Abtrennung von Biogas 11 aus der methanogenen mikrobiellen Umsetzung hervorgeht. Generell wird erfindungsgemäß Biomasse 10 zu Biogas 11 und einem wässrigen Biomasserückstand 12 umgesetzt. Die zweite Fermenterbrühe, in der die Biogasfermentation abläuft, die auch als Biogasfermenterbrühe bezeichnet ist, und der wässrige Biomasserückstand 12 enthalten NH₃, das daraus abgetrennt und der Milchsäurefermentation zugeführt wird. Für diese Biogaserzeugung aus Biomasse 10 eignen sich bekannte methanogene, insbesondere thermophile Fermentationsverfahren, bei denen als Biomasse 10 stickstoffhaltige Verbindungen, insbesondere tierische Exkremente, insbesondere aus der Schweine- und Geflügelhaltung anfallende Exkremente verwendet werden können. Das bei der methanogenen mikrobiellen Umsetzung von Biomasse 10, die Protein, Harnsäure und/oder Harnstoff enthält, entstehende NH₃ 13 kann beispielsweise durch Ausstrippen eines Teils der zweiten Biogasfermenterbrühe und/oder des wässrigen Biomasserückstands 12 mit einem Strippmedium 18 erfolgen, vorzugsweise durch Ausstrippen aus der Flüssigphase 12b, die durch Abtrennung der Festphase 12a aus wässrigen Biomasserückstand 12 erzeugt ist. Wässriger Biomasserückstand 12, vorzugsweise die daraus abgetrennte Flüssigphase 12b, wird bevorzugt mit Lauge versetzt, um ihren pH anzuheben, beispielsweise auf pH 7-12, vorzugsweise pH 7- 9, wobei als Lauge beispielsweise Natriumhydroxid zugesetzt werden kann. Die Separation wässrigen Biomasserückstands 12 in Festphase 12a und Flüssigphase 12b kann wahlweise kontinuierlich oder satzweise mit einem Volumenanteil erfolgen, der aus der Biogasfermenterbrühe, d.h. der gärenden Biomasse 10 bzw. dem wässrigen Biomasserückstand 12 entnommen wird. Die Flüssigphase 12b des wässrigen Biomasserückstands 12 bzw. die Biogasfermenterbrühe kann nach Abtrennung zumindest eines Teils des NH₃ 13 rückgeführt werden, beispielsweise in die Biomasse 10, in die Biogasfermenterbrühe oder in den wässrigen Biomasserückstand 12. Die Abtrennung von NH₃ 13 erfolgt vorzugsweise durch Strippen des wässrigen Biomasserückstands 12 oder von dessen Flüssigphase 12b mit einem Trägergas 18, wobei z.B. Trägergas 18, das insbesondere Abluft aus dem ersten Gärungsbehälter 21 ist, als Strippmedium in eine Strippeinrichtung 33 eingeleitet wird. Abgetrenntes bzw. ausgestripptes NH₃ 13 kann als eine gasförmige Phase 14 mit einem Gehalt an NH₃ gesammelt und z.B. komprimiert oder als NH₃ kondensiert werden, und wahlweise kann aus dem NH₃ 13 eine wässrige Phase 15 ausgewaschen werden, wobei eine gasförmige, NH₃-haltige Phase 16 zurückbleibt. Vorzugsweise ist der gasförmige Anteil 14 der ausgestrippten Phase 13 im wesentlichen gasförmiges NH₃, das durch Verdichten und/oder Lösen in Wasser verarbeitet werden kann. Eine durch Waschen von ausgestripptem NH₃ 13 erzeugte Gasphase 16, von der optional NH₃ 14 durch Kompression oder Kondensation teilweise abgetrennt ist, weist noch einen Gehalt an NH₃ auf und kann als NH₃-haltige Gasphase 16 der ein erstes Substrat, insbesondere Lactose und/oder der Lactat enthaltenden ersten Fermenterbrühe 1, 2 zugesetzt werden. Eine beim Waschen von ausgestripptem NH₃ anfallende wässrige Phase 15 kann als wässriges Ammoniak 15' entnommen werden.

Generell ist es bevorzugt, NH₃ aus einem Teilstrom abzutrennen, insbesondere durch Strippen, wobei der Teilstrom vorzugsweise kontinuierlich aus der Biogasfermenterbrühe oder aus der von dem wässrigen Biomasserückstand abgetrennten Flüssigphase 12b entnommen wird und nach dem Abtrennen von NH₃ zur Biogasfermenterbrühe rückgeführt wird. Besonders bevorzugt werden vor dem Abtrennen bzw. Strippen Feststoffe aus dem Teilstrom abgetrennt, z.B. mittels eines Absetzbehälters und/oder mittels eines kontinuierlichen Dekanters.

Für Abtrennung von NH₃ wird der pH der Biogasfermenterbrühe bzw. einer daraus abgetrennten flüssigen Phase vorzugsweise auf einen Wert von ca. 10 bis 11,5, bevorzugter pH 10,5 bis 11 eingestellt, z.B. durch Zugabe von Lauge.

Weiter bevorzugt ist es, für das Strippen die Flüssigphase auf mindestens ca. 50 °C bis mindestens ca. 65 °C zu temperieren, z.B. mittels Wärme, die aus dem Gärungsbehälter der Biogasfermentation abgeführt wurde, oder mittels Oxidation von Biogas erzeugter Wärme. Das Temperieren der Flüssigphase kann z.B. durch Temperierung der Strippeinrichtung mit der Wärme erfolgen. Weiter optional zusätzlich oder alternativ zu dieser Temperierung kann die Strippeinrichtung bei Unterdruck betrieben werden, so dass NH₃ aus dem aus der Strippeinrichtung austretenden Gas durch Kühlung und/oder Druckerhöhung, z.B. auf Normaldruck oder darüber, abgetrennt werden kann, z.B. durch Kondensation und/oder mittels Gaswäsche, vorzugsweise mit Wasser.

NH₃ 13 kann daher der ein erstes Substrat, insbesondere Lactose- und/oder Lactat-haltigen ersten Fermenterbrühe 1, 2 unmittelbar als Teil der abgetrennten, vorzugsweise ausgestrippten, NH₃-haltigen Phase 13 zugeführt werden, und ein Teil der NH₃-haltigen Phase 13 kann durch Kompression und/oder Kondensation als gasförmige Phase 14 abgetrennt werden und/oder aus der NH₃-haltigen Phase 13 kann durch Waschen eine wässrige Phase 15 und eine Gasphase 16 erzeugt werden. Das NH₃, das der Milchsäurefermentation zugeführt wird, kann jeder dieser Fraktionen entstammen, da alle diese Fraktionen NH₃ aufweisen, das aus der mikrobiellen methanogenen Umsetzung von Biomasse 10 zu wässrigem Biomasserückstand 12 stammt.

Insbesondere bei Verwendung von NH₃ in gasförmiger Form 13, 14 und/oder 16 kann unlösliches Gas, das in die Lactose und/oder Lactat enthaltende Fermenterbrühe 1, 2 eingetragen wird, nach Durchströmen der Fermenterbrühe als Abluft 17 gesammelt werden und wahlweise als Strippmedium 18 zum Durchströmen des wässrigen Biomasserückstands 12, vorzugsweise der Flüssigphase 12b des Biomasserückstands 12 für das Ausstrippen von NH₃ 13 verwendet werden, z.B. als Strippmedium in die Strippeinrichtung 33 eingeleitet werden kann. Auf diese Weise verwendet das integrierte Herstellungsverfahren zumindest einen Teil der Abluft 17 im Kreislauf als Strippmedium 18 und vermeidet zumindest einen Teil von Immissionen.

Beim erfindungsgemäßen Verfahren ist die Milchsäurefermentation auch energetisch mit der methanogenen Biomassefermentation gekoppelt. Dabei kann die Wärme 19, die bei der mikrobiellen methanogenen Umsetzung von Biomasse 10 in der zweiten Fermenterbrühe entsteht und für eine optimale Erzeugung von Biogas 11 kontinuierlich daraus abgeführt wird, für die Erwärmung der Lactose enthaltenen ersten Fermenterbrühe 1 und/oder der Lactat enthaltenen ersten Fermenterbrühe 2 verwendet werden. In dieser Ausführungsform entfällt daher der zusätzliche Aufwand für die Erwärmung der ein Substrat, vorzugsweise ein Saccharid, insbesondere Lactose und/oder Lactat enthaltenden ersten Fermenterbrühe 1, 2 der Milchsäurefermentation, da die Abwärme 19 aus der Biogasfermentation von Biomasse 10 verwendet wird. Alternativ oder zusätzlich zur Verwendung der Abwärme 19 kann auch Wärme 20, die bei der Oxidation von Biogas 11 entsteht, zur Erwärmung der ein Substrat, z.B. ein Saccharid, insbesondere Lactose, enthaltenden oder Lactat enthaltenen ersten Fermenterbrühe 1, 2 verwendet werden. Für die Oxidation zur Erzeugung von Wärme 20 kann beispielsweise ein mit Biogas 11 betriebener Gasmotor verwendet werden, wie er beispielsweise für die Stromerzeugung mit einem Generator eingesetzt wird, oder Biogas 11 kann für die Beheizung von Lactose oder Lactat enthaltener Fermenterbrühe 1, 2 verbrannt werden.

Weiterhin zeigt die Figur schematisch die Elemente der Vorrichtung, die zur Durchführung des erfindungsgemäßen Verfahrens Verwendung finden kann. Ein erster Gärungsbehälter 21, der für die mikroaerophile bzw. anaerobe Fermentation einer wässrigen Saccharidlösung, insbesondere einer Lactoselösung 1 mittels Lactat produzierender Mikroorganismen eingerichtet ist, ist mit einer Entnahmeleitung 22 zur Entnahme von Lactat enthaltender Fermenterbrühe 2 versehen, die beispielsweise mit einem Teilvolumen der Fermenterbrühe 2 durchströmt ist und vorzugsweise mit einer die Fermenterbrühe 2 aufnehmenden Trenneinrichtung 23 verbunden ist. Die Trenneinrichtung 23 kann beispielsweise eine Elektrodialyseeinrichtung, eine Ultrafiltrationseinrichtung und/oder ein Absetzbehälter bzw. ein Separator sein. Die Trenneinrichtung 23 kann beispielsweise zur Abtrennung von Ammoniumlactat 3 dienen, oder zur Ausfällung von Calcium-Ammoniumlactat 5, insbesondere nach Zugabe eines Calciumsalzes, oder für die Abtrennung zumindest eines Teils der Mikroorganismen 4 eingerichtet sein.

Die stoffliche Kopplung der Milchsäurefermentation mit der Biogasfermentation erfolgt erfindungsgemäß dadurch, dass NH₃ 13, das vorzugsweise durch Strippen aus dem wässrigen Biomasserückstand 12 oder dessen wässriger Phase 12b abgetrennt ist, beispielsweise in Form der komprimierten und/oder kondensierten gasförmigen Phase 14, der durch Gaswäsche aus abgetrenntem NH₃ 13, das insbesondere in einer Strippeinrichtung 33 ausgestrippt ist, hergestellten wässrigen Phase 15 oder deren Gasphase 16 durch Leitungen 24, die mit dem ersten Gärungsbehälter 21 und/oder der Trenneinrichtung 23 verbunden sind, in die Lactat enthaltende Fermenterbrühe 2 eingebracht wird.

NH₃ 13 wird erfindungsgemäß durch methanogene mikrobielle Umsetzung von Biomasse 10 in einem zweiten Gärungsbehälter 31 erzeugt, der für die Biogasfermentation eingerichtet ist, aus dem wässriger Biomasserückstand 12 abgeführt werden kann, und vorzugsweise mittels einer Trenneinrichtung 32, beispielsweise eines Separators oder Absetzbeckens, in eine Flüssigphase 12b und eine Festphase 12a aufgetrennt wird. Die Flüssigphase 12b wird vorzugsweise in einen Strippbehälter oder eine Strippeinrichtung 33 geleitet, um dort durch Zugabe eines Strippmediums 18 NH₃ 13 auszustrippen, während eine von NH₃ 13 abgereicherte Fraktion der flüssigen Rückstandsphase verbleibt. Die Strippeinrichtung 33 ist vorzugsweise mit einer Zugabeeinrichtung für Lauge 34 versehen, um den pH auf zumindest pH 7 anzuheben.

Die NH₃-haltige Phase 13, vorliegend auch als ausgestrippte Phase 13 bezeichnet, daraus abgetrenntes, vorzugsweise komprimiertes oder kondensiertes gasförmiges NH₃ 14, oder aus der abgetrennten NH₃-haltigen Phase 13 durch Waschen erzeugte wässrige Phase 15 und Gasphase 16, die jeweils einen Anteil von NH₃ 13 aufweisen, können in bevorzugter Ausführungsform jeweils in separaten Behältern 35 zwischengepuffert werden.

Vorzugsweise weist eine Leitung 24, die NH₃ 13 aus gasförmiger Phase 14, durch Waschen erzeugter wässriger Phase 15 und/oder gewaschener Gasphase 16 zu dem ersten Gärungsbehälter 21 und/oder zur Trenneinrichtung 23 leitet, zumindest einen Pufferbehälter 35 auf. Der zumindest eine Pufferbehälter 35 ist vorzugsweise mit einer gemeinsamen oder separaten Ein- und Auslassöffnung versehen, die durch jeweils ein Ventil geschlossen werden kann. Auf diese Weise kann ein Pufferbehälter 35 für die stoffliche Kopplung der Biogasfermentation mit der Milchsäurefermentation verwendet werden, um NH₃ 13, das gasförmig oder in wässriger Lösung vorliegen kann, von der Einrichtung zur Biogasfermentation, beispielsweise von einem der Behälter, die NH₃ 13, daraus hergestellte gasförmige NH₃-Phase 14, durch Waschen erhaltene wässrige Phase 15 oder Gasphase 16 enthalten, für den Transport geschlossen werden, und anschließend an den ersten Gärungsbehälter 21 oder an die Trenneinrichtung 23 angeschlossen werden.

Weiter weist die erfindungsgemäße Vorrichtung eine Wärmeübertragungseinrichtung 36 auf, mit der Wärme 19, die aus dem zweiten Gärungsbehälter 31 abgeführt wird, zum ersten Gärungsbehälter 21 transportiert werden, oder zu einem Mischbehälter 37, in dem die Lactat enthaltende Fermenterbrühe 1 angesetzt wird.

Die Fraktion der ersten Fermenterbrühe 2, von der Ammoniumlactat abgetrennt wurde, kann Mikroorganismen, insbesondere Lactobazillen 4, und nicht verstoffwechselte Bestandteile des ersten Substrats enthalten. Diese Fraktion wird, optional nach Zwischenspeicherung in einem Pufferbehälter 38, in den zweiten Gärungsbehälter 31 geleitet, so dass aus den aus der ersten Fermenterbrühe abgetrennte Mikroorganismen und Bestandteile des ersten Substrats zu Biogas umgesetzt werden können.

### Beispiel: Fermentative Herstellung von Ammoniumlactat

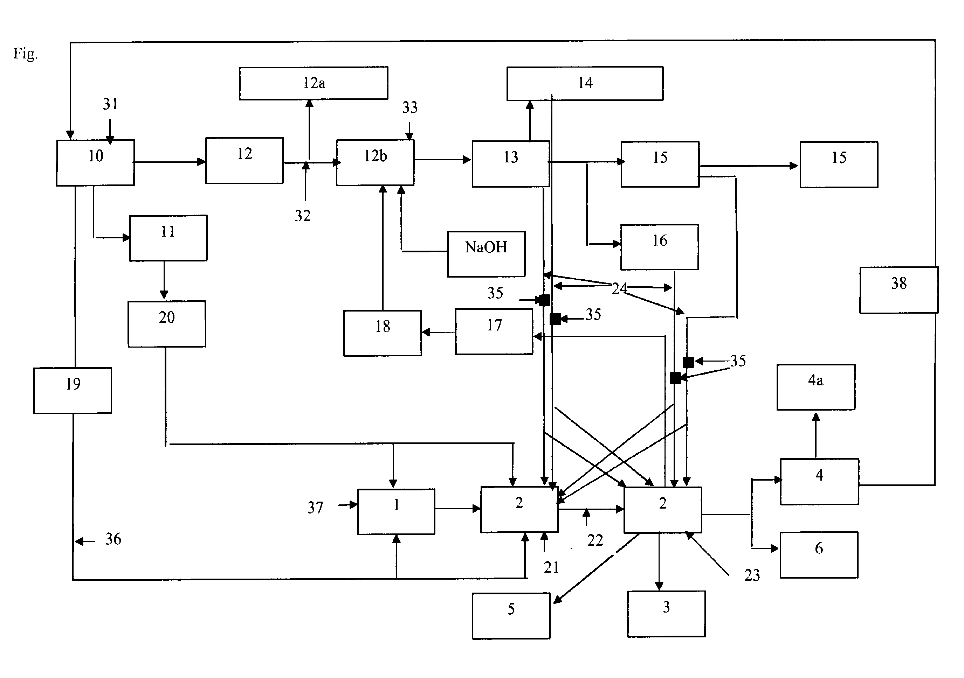

In dem Verfahren, das schematisch in Figur 1 gezeigt ist, wurde deproteinierte Molke in einem ersten Gärungsbehälter unter Zusatz von Lactobazillen zu Milchsäure fermentiert. Der Behälter wies ein Arbeitsvolumen von 3 m³ auf. Zur Einstellung des pH-Werts wurde ein NH₃-Luftgemisch zugegeben; der pH wurde auf einen Wert von ca. 7 eingeregelt.

Der Gärungsbehälter wurde mit einem Doppelmantel auf eine Temperatur von ca. 37 °C erwärmt.

Eine in der Nähe angeordnete Anlage zur Biogasfermentation wies einen zweiten Gärungsbehälter 31 auf, der mit Gülle aus einem Schweinemastbetrieb beschickt wurde, die als Biomasse eingesetzt wurde. Dieser Gärungsbehälter 31 wurde mit Wasser gekühlt, das für die Erwärmung des ersten Gärungsbehälters 21 eingesetzt wurde, der für die Molkefermentation verwendet wurde.

Aus dem zweiten Gärungsbehälter 31 der Biomassefermentation abgetrenntes Biogas wurde in einem Gasmotor oxidiert, der an einen Stromgenerator gekoppelt war. Aus dem zweiten Gärungsbehälter 31 wurde kontinuierlich ein Volumenstrom entnommen und rückgeführt, von dem eine Flüssigphase mittels eines Absetzbeckens abgetrennt wurde. Abhängig vom Gehalt an Feststoffen wurde der wässrige Biomasserückstand 12, bzw. die Flüssigphase 12 durch einen kontinuierlichen Dekanter (z.B. CB 505, erhältlich von Westfalia Separator Umwelttechnik) von Feststoffen im Wesentlichen befreit, optional wurde vor dem Dekantieren Flockungsmittel zugesetzt. Der pH-Wert der Flüssigphase lag bei ca. 8 bis 8,5 und wurde vorzugsweise mittels Zugabe von Lauge (50 Gew.-% Natronlauge) auf pH 10,5 bis 11 angehoben. Durch die Alkalisierung verursachte Ausfällungen wurden nicht beobachtet. Für das Strippen wurde die Flüssigphase 12b auf ca. mindestens 50 bis 55 °C temperiert, z.B. mittels Wärme, die aus dem Gärungsbehälter der Biogasfermentation abgeführt wurde, oder durch Oxidation von Biogas in einem Gasmotor, wobei dessen Kühlwasser zur Temperierung der Strippeinrichtung verwendet wurde. Diese Flüssigphase 12b wurde in der Strippeinrichtung 33 mit Luft durchströmt, sodass NH₃ ausgestrippt wurde.

Als Strippeinrichtung 33 wurde, wie generell für die Erfindung geeignet, eine Füllkörperkolonne als Strippkolonne eingesetzt. Die Strippkolonne konnte bei Umgebungsdruck betrieben werden, wobei am Sumpf wasserdampfgesättigte Luft zugeführt wurde und am Kolonnenkopf die alkalisierte Flüssigphase. Am Kolonnenkopf wurde die ammoniakhaltige Luft abgeführt. Vom anfänglichen Gehalt der Flüssigphase von ca. 8 g/L NH₃ konnten ca. 5 bis 6 g/L NH₃ ausgestrippt werden.

Die vom Kolonnenkopf abgeführte und mit NH₃ versehene Luft wurde unmittelbar dem ersten Gärungsbehälter 21 der Milchsäurefermentation zugeführt, wobei ein Filter zur Abtrennung von Aerosol in der Leitung angeordnet war. Entsprechend der generell bevorzugten Ausführungsform wurde Gas bzw. Luft, die aus dem Gärungsbehälter 21 der Milchsäurefermentation abgeführt wurde, als Strippmedium der Strippkolonne 33 zugeführt.

Alternativ, z.B. zur Verwendung bei einer größeren räumlichen Entfernung der Biogasfermentationsanlage von der Milchsäurefermentation wurde eine Ausführungsform der Erfindung dadurch nachgestellt, dass die aus der Strippeinrichtung 33 austretende NH₃-haltige Luft in einen verschließbaren Druckbehälter gesammelt wurde, der mittels eines Ventils verschlossen und von der Strippeinrichtung getrennt werden konnte. Der geschlossene Druckbehälter wurde zum ersten Gärungsbehälter 21 der Milchsäurefermentation transportiert und an diesen angeschlossen, wobei die Zudosierung der NH₃-haltigen Luft mittels der pH-Kontrolle gesteuert wurde.

Die Milchsäurefermentation wurde vorzugsweise im Fed-batch-Verfahren durchgeführt, und nach Erreichen des Maximalvolumens, oder dann, wenn keine reduzierenden Zucker mehr in der Fermenterbrühe nachgewiesen werden konnten, wurde die Fermenterbrühe anteilig zur Elektrodialyse eingesetzt, und es konnte eine wässrige Lösung von Ammoniumlactat erhalten werden. Feststoffe, insbesondere Mikroorganismen, wurden vorzugsweise vor der Elektrodialyse mittels eines Separators abgetrennt.

Ein Aliquot der Ammoniumlactat enthaltenden Fermenterbrühe wurde mit Calciumsulfat oder Calciumchlorid versetzt, und es konnte, optional nach Entfernen eines Teils des Wassers, Calcium-Ammoniumlactat ausgefällt werden.

Es hat sich gezeigt, dass entweder nach Abtrennung von Ammoniumlactat, beispielsweise mittels Elektrodialyse, vorzugsweise mit vorheriger Filtration zur Abtrennung von Milchsäurebakterien, ein probiotisches Präparat erzeugt werden konnte, das insbesondere zur Verwendung als Tierfutter findet.

Bei Verwendung von stärkefreiem Kartoffelfruchtwasser oder Erbsenfruchtwasser als erstes Substrat für die Milchsäurefermentation konnten die darin enthaltenen löslichen Saccharide zumindest anteilig zu Milchsäure umgesetzt werden, während die Peptide das Wachstum von Lactobazillen erlaubten. Vorzugsweise wurde zusätzlich Molke und/oder Melasse als Bestandteil des erstes Substrat

Die Fraktion der ersten Fermenterbrühe der Milchsäurefermentation, die nach Abtrennung von Ammoniumlactat erhalten wurde, und optional von der ersten Fermenterbrühe abgetrennte Mikroorganismen wurden dem zweiten Gärungsbehälter 31 zugeführt. Die in der Fraktion enthaltenen Bestandteile des ersten Substrats, z.B. Protein aus stärkefreiem Kartoffel- oder Erbsenfruchtwasser, und die Mikroorganismen führten nach Zuführung in die Biogasfermentation zu einer Steigerung der Biogasproduktion.

### Bezugszeichenliste:

1 Lactose enthaltende Fermenterbrühe
2 Lactat enthaltende Fermenterbrühe
3 Ammoniumlactat
4 Lactobazillen
4a probiotisches Präparat
5 Calcium-Ammoniumlactat
6 Milchsäure
10 Biomasse
11 Biogas
12 wässriger Biomasserückstand
12a Festphase Biomasserückstand
12b Flüssigphase Biomasserückstand
13 NH3
14 gasförmige Phase
15 wässrige Phase
15' wässriges Ammoniak
16 Gasphase
17 Abluft
18 Strippmedium
19 Wärme
20 Wärme
21 erster Gärungsbehälter
22 Entnahmeleitung
23 Trenneinrichtung
24 Leitungen
31 zweiter Gärungsbehälter
32 Trenneinrichtung
33 Strippbehälter oder Strippeinrichtung
34 Zugabeeinrichtung
35 Pufferbehälter
36 Wärmeübertragungseinrichtung
37 Mischbehälter
38 Pufferbehälter

## Patentansprüche

1. Verfahren zur Herstellung von Ammoniumlactat durch mikrobielle Umsetzung einer ein erstes Substrat enthaltenden wässrigen Fermenterbrühe (1) zu einer Lactat enthaltenden ersten Fermenterbrühe (2) mit dem Schritt der Zugabe von NH₃ (13) zu der ersten Fermenterbrühe (2) und Abtrennung von Ammoniumlactat von einer Fraktion der ersten Fermenterbrühe (2), **dadurch gekennzeichnet, dass** das der Lactat enthaltenden ersten Fermenterbrühe (2) zugegebene NH₃ (13) erzeugt wird durch methanogene mikrobielle Umsetzung von stickstoffhaltigem Substrat mit oder aus Biomasse (10), die Protein, Harnsäure und/oder Harnstoff enthält, zu einem Biogas (11) mit einem Gehalt an Methan und einer NH₃-haltigen zweiten Biogasfermenterbrühe und Abtrennen von NH₃ (13) aus der NH₃-haltigen zweiten Biogasfermenterbrühe,
wobei der die Biomasse (10) enthaltenden zweiten Biogasfermenterbrühe während der methanogenen Umsetzung mittels eines Wärmetauschermediums Wärme entzogen wird und die Lactat enthaltende erste Fermenterbrühe (2) mit dieser Wärme erwärmt wird und
die Fraktion der ersten Fermenterbrühe (2), von der Ammoniumlactat abgetrennt ist, zumindest anteilig der zweiten Fermenterbrühe zugeführt wird.

2. Verfahren nach Anspruch 1, **dadurch gekennzeichnet, dass** die Abtrennung von NH₃ (13) durch Einstellen eines neutralen oder alkalischen pH-Werts und Ausstrippen einer NH₃-haltigen Phase (14) mittels eines Strippmediums aus der zweiten Biogasfermenterbrühe oder aus einem wässrigen Biomasserückstand (12) erfolgt, der von der zweiten Biogasfermenterbrühe abgetrennt ist.

3. Verfahren nach Anspruch 2, **dadurch gekennzeichnet, dass** die Abluft aus der ersten Fermenterbrühe (2) das Strippmedium (18) ist und in die zweite Biogasfermenterbrühe oder in den wässrigen Biomasserückstand (12) oder in eine daraus abgetrennte Flüssigphase eingeleitet wird.

4. Verfahren nach Anspruch 2 oder 3, **dadurch gekennzeichnet, dass** das der ersten Fermenterbrühe (2) zugegebene NH₃ (13) gasförmig und/oder die durch das Ausstrippen erzeugte NH₃-haltigen Phase (14) ist.

5. Verfahren nach einem der Ansprüche 2 bis 4, **dadurch gekennzeichnet, dass** die ausgestrippte NH₃-haltige Phase (14) mit Wasser kontaktiert wird und die erhaltene wässrige Phase (15) von der erhaltenen Gasphase (16) getrennt wird, wobei die wässrige Phase (15) oder die erhaltene Gasphase (16) das der ersten Fermenterbrühe (2) zugegebene NH₃ (13) ist.

6. Verfahren nach einem der voranstehenden Ansprüche, **dadurch gekennzeichnet, dass** die erste Fermenterbrühe (1, 2) als erstes Substrat Stärke, Protein, Molke, deproteinierte Molke, verteiltes Molkepulver, Kartoffelpulpe, Erbsenpulpe, stärkefreies Kartoffelfruchtwasser, stärkefreies Erbsenfruchtwasser und/oder Melasse enthält oder daraus besteht.

7. . Verfahren nach einem der voranstehenden Ansprüche, durch gekennzeichnet, dass Ammoniumlactat mittels Ultrafiltration und/oder Elektrodialyse aus der Lactat enthaltenden ersten Fermenterbrühe (2) abgetrennt wird.

8. Verfahren nach einem der voranstehenden Ansprüche, **dadurch gekennzeichnet, dass** die Lactat enthaltende erste Fermenterbrühe (2) mit einem Calciumsalz versetzt wird und Ammoniumlactat als Calcium-Ammoniumlactat (5) aus der ersten Fermenterbrühe (2) abgetrennt wird.

9. Verfahren nach einem der voranstehenden Ansprüche, **dadurch gekennzeichnet, dass** ein Anteil der ersten Fermenterbrühe (2) vor oder nach Abtrennung von Ammoniumlactat durch Entzug von Wasser zu einer Bakterien enthaltenen Zusammensetzung aufkonzentriert oder getrocknet wird, um ein probiotische Bakterien enthaltendes Präparat (4a) zu erzeugen.

10. Verfahren nach einem der voranstehenden Ansprüche, **dadurch gekennzeichnet, dass** zumindest ein Anteil des erzeugten Methans oxidiert wird und die erste Fermenterbrühe mit Wärme, die bei der Oxidation erzeugt wird, temperiert wird.

11. Vorrichtung zur Herstellung von Ammoniumlactat mit einem ersten Gärungsbehälter (21), der zur Aufnahme einer wässrigen, ein erstes Substrat für eine Milchsäuregärung enthaltende Lösung (1) eingerichtet ist und der eine erste Zuleitung (24) für gasförmiges NH₃ (13) und eine Entnahmeleitung (22) zur Entnahme von Lactat enthaltender erster Fermenterbrühe (2) aufweist, **dadurch gekennzeichnet, dass** die erste Zuleitung (24) mit einer mit einem zweiten Gärungsbehälter (31) verbundenen Auslassleitung für NH₃ (13) verbunden ist und zwischen dem zweiten Gärungsbehälter (31) und der ersten Zuleitung (24) eine Strippeinrichtung (33) zum Ausstrippen von NH₃ angeordnet ist und die Strippeinrichtung (33) eine Zuleitung für Strippmedium aufweist, die mit einer Abluftleitung des ersten Gärungsbehälters (21) verbunden ist, wobei der zweite Gärungsbehälter (31) für die methanogene mikrobielle Umsetzung von Protein, Harnsäure und/oder Harnstoff enthaltender Biomasse (10) zu einem Biogas (11) mit einem Gehalt an Methan und einer NH₃-haltigen zweiten Biogasfermenterbrühe eingerichtet ist, und der erste Gärungsbehälter (21) einen ersten Wärmetauscher aufweist, der mittels einer Zu- und einer Ableitung für Wärmetauschermedium mit einem zweiten Wärmetauscher gekoppelt ist, der am zweiten Gärungsbehälter (31) angeordnet ist.

12. Vorrichtung nach Anspruch 11, **dadurch gekennzeichnet, dass** zwischen dem zweiten Gärungsbehälter (31) und der ersten Zuleitung (24) ein Separator mit einem Auslass für eine Flüssigphase und einem Auslass für eine Festphase angeordnet ist, wobei die Strippeinrichtung (33) am Auslass für die Flüssigphase angeschlossen ist.

13. Vorrichtung nach einem der Ansprüche 11 bis 12, **dadurch gekennzeichnet, dass** die Auslassleitung einen Pufferbehälter (35) zur Aufnahme von NH₃ (13), das gasförmig oder in wässriger Lösung vorliegt, aufweist.

14. Vorrichtung nach einem der Ansprüche 11 bis 13, **dadurch gekennzeichnet, dass** mit der Entnahmeleitung (22) zur Entnahme von Lactat enthaltender erster Fermenterbrühe (2) eine Trenneinrichtung (23) verbunden ist, welche zur Abtrennung von Ammoniumlactat aus der ersten Fermenterbrühe (2) eingerichtet ist und die eine Leitung zur Zuführung der Fraktion der ersten Fermenterbrühe (2) in den zweiten Gärungsbehälter (31) aufweist, von der Ammoniumlactat abgetrennt ist.

15. Vorrichtung nach einem der Ansprüche 11 bis 14, **dadurch gekennzeichnet, dass** die erste Zuleitung (24) für gasförmiges NH₃ (13) mit dem Auslass der Strippeinrichtung (33) für NH₃-haltiges Gas verbunden ist.

## Claims

1. Process for the production of ammonium lactate by microbial transformation of an aqueous fermentation broth (1) containing a first substrate to a first fermentation broth (2) containing lactate with the step of adding NH₃ (13) to the first fermentation broth (2) and separation of ammonium lactate from a fraction of the first fermentation broth (2), **characterized in that** the NH₃ (13) added to the first fermentation broth (2) containing the lactate is produced by methanogenic microbial transformation of nitrogen-containing substrate with or from biomass (10) containing protein, uric acid and/or urea to a biogas (11) having a content of methane and to a second biogas fermentation broth containing NH₃, and separation of NH₃ (13) from the second biogas fermentation broth containing NH₃, wherein during the methanogenic transformation thermal energy is removed by means of a heat exchanger medium from the second biogas fermentation broth containing the biomass (10), and the first fermentation broth (2) containing lactate is heated with this thermal energy, and the fraction of the first fermentation broth (2) from which ammonium lactate has been separated is at least partially added to the second fermentation broth.

2. Process according to claim 1, **characterized in that** the separation of NH₃ (13) occurs by adjusting a neutral or alkaline pH value and stripping of a NH₃ containing phase (14) by means of a stripping medium from the second biogas fermentation broth or from an aqueous biomass residue (12) which has been separated from the second biogas fermentation broth.

3. Process according to claim 2, **characterized in that** the exhaust air from the first fermentation broth (2) is the stripping medium (18) and is introduced into the second biogas fermentation broth or into the aqueous biomass residue (12) or into a liquid phase separated therefrom.

4. Process according to claim 2 or 3, **characterized in that** the NH₃ (13) added to the first fermentation broth (2) is gaseous and/or is the NH₃ containing phase (14) produced by the stripping.

5. Process according to one of the claims 2 to 4, **characterized in that** the stripped NH₃ containing phase (14) is contacted with water and the resulting aqueous phase (15) is separated from the resulting gaseous phase (16), wherein the aqueous phase (15) or the resulting gaseous phase (16) is the NH₃ (13) added to the first fermentation broth (2).

6. Process according to one of the preceding claims, **characterized in that** the first fermentation broth (1, 2) as first substrate contains or consists of starch, protein, whey, de-proteinised whey, distributed whey powder, potato pulp, pea pulp, starch-free swelling water from potatoes, starch-free swelling water from peas and/or molasses.

7. Process according to one of the preceding claims, **characterized in that** ammonium lactate is removed from the lactate containing first fermentation broth (2) by ultrafiltration and/or by electrodialysis.

8. Process according to one of the preceding claims, **characterized in that** a calcium salt is added to the first fermentation broth (2) containing lactate and ammonium lactate is separated from the first fermentation broth (2) as calcium ammonium lactate.

9. Process according to one of the preceding claims, **characterized in that** prior to or after the separation of ammonium lactate a portion of the first fermentation broth (2) is concentrated or dried to a bacteria containing composition by extraction of water, in order to produce a probiotic bacteria containing preparation (4a).

10. Process according to one of the preceding claims, **characterized in that** at least a portion of the methane produced is oxidized and the first fermentation broth is thermostated using thermal energy which is produced by the oxidation.

11. Device for the production of ammonium lactate having a first fermentation container (21) which is disposed for receiving an aqueous solution (1) containing a first substrate for a lactic fermentation and which has a first feeding pipe (24) for gaseous NH₃ (13) and a removal duct (22) for removal of first fermentation broth (2) containing lactate, **characterized in that** the first feeding pipe (24) is connected to an outlet duct for NH₃ (13) which is connected to a second fermentation container (31), and between the second fermentation container (31) and the first feeding pipe (24) a stripping device (33) for stripping of NH₃ is arranged the stripping device having a feeding pipe for stripping medium which is connected to an exhaust air duct of the first fermentation container (21), wherein the second fermentation container (31) is disposed for the methanogenic microbial transformation of biomass (10) containing protein, uric acid and/or urea to a biogas (11) having a content of methane and to a second biogas fermentation broth containing NH₃, and the first fermentation container (21) has a first heat exchanger coupled to a second heat exchanger by means of a feeding pipe and a discharge pipe for heat exchanging medium, which second heat exchanger is arranged at the second fermentation container (31).

12. Device according to claim 11, **characterized in that** a separator having an outlet for a liquid phase and an outlet for a solid phase is arranged between the second fermentation container (31) and the first feeding pipe (24), wherein the stripping device (33) is connected to the outlet for the liquid phase.

13. Device according to one of the claims 11 to 12, **characterized in that** the outlet duct has a buffer container (35) for intake of NH₃ (13) which is gaseous or in aqueous solution.

14. Device according to one of the claims 11 to 13, **characterized in that** a separation device (23) is connected to the removal duct (22) for removal of first fermentation broth (2) containing lactate, which separation device is disposed for removal of ammonium lactate from the first fermentation broth (2) and has a duct for addition of the fraction of the first fermentation broth (2) into the second fermentation container (31) from which ammonium lactate has been separated.

15. Device according to one of the claims 11 to 14, **characterized in that** the first feeding pipe (24) for gaseous NH₃ (13) is connected to the outlet of the stripping device (33) for gas containing NH₃.

## Revendications

1. Procédé pour produire du lactate d'ammonium par transformation microbienne d'un bouillon de fermentation (1) aqueux contenant un premier substrat en un premier bouillon de fermentation (2) contenant du lactate, comportant l'étape qui consiste à ajouter NH₃ (13) au premier bouillon de fermentation (2) et à séparer le lactate d'ammonium d'une fraction du premier bouillon de fermentation (2), **caractérisé en ce que**
le NH₃ (13) ajouté au premier bouillon de fermentation (2) contenant le lactate est produit par transformation microbienne méthanogène d'un substrat contenant de l'azote comportant ou provenant de la biomasse (10) qui contient du protéine, de l'acide urique et/ou de l'urée en un biogaz (11) contenant du méthane et en un deuxième bouillon de fermentation de biogaz contenant NH₃ et NH₃ (13) est séparé du deuxième bouillon de fermentation de biogaz contenant NH₃,
de la chaleur est extraite, à l'aide d'un agent d'échange thermique, du deuxième bouillon de fermentation de biogaz contenant la biomasse (10) pendant la transformation méthanogène et le premier bouillon de fermentation (2) contenant le lactate est chauffé par cette chaleur et
la fraction du premier bouillon de fermentation (2) de laquelle est séparé le lactate d'ammonium est conduite au moins en partie au deuxième bouillon de fermentation.

2. Procédé selon la revendication 1, **caractérisé en ce que** la séparation de NH₃ (13) est réalisée en réglant une valeur de pH neutre ou basique et en strippant à l'aide d'un agent de strippage une phase (14) contenant NH₃ du deuxième bouillon de fermentation de biogaz ou d'un résidu de biomasse (12) aqueux qui est séparé du deuxième bouillon de fermentation de biogaz.

3. Procédé selon la revendication 2, **caractérisé en ce que** l'air provenant du premier bouillon de fermentation (2) est l'agent de strippage (18) et qu'il est conduit dans le deuxième bouillon de fermentation de biogaz ou dans le résidu de biomasse (12) aqueux ou dans une phase liquide qui en est séparée.

4. Procédé selon la revendication 2 ou 3, **caractérisé en ce que** le NH₃ (13) ajouté au premier bouillon de fermentation (2) est gazeux et/ou qu'il est la phase (14) contenant NH₃ obtenue par strippage.

5. Procédé selon l'une des revendications 2 à 4, **caractérisé en ce que** la phase (14) strippée contenant NH₃ est mise en contact avec de l'eau et que la phase aqueuse (15) obtenue est séparée de la phase gazeuse (16) obtenue, la phase aqueuse (15) ou la phase gazeuse (16) obtenue étant le NH₃ (13) ajouté au premier bouillon de fermentation (2).

6. Procédé selon l'une des revendications précédentes, **caractérisé en ce que** le premier bouillon de fermentation (1, 2) sous forme de premier substrat est l'amidon, une protéine, le petit-lait, le petit-lait déprotéinisé, la poudre de petit-lait distribué, la pâte de pommes de terre, la pâte de pois, le lait de fécule de pommes de terre sans la fécule, le lait de fécule de pois sans la fécule et/ou la mélasse ou en contient.

7. Procédé selon l'une des revendications précédentes, **caractérisé en ce que** le lactate d'ammonium est séparé du premier bouillon de fermentation (2) contenant du lactate par ultrafiltration et/ou par électrodialyse.

8. Procédé selon l'une des revendications précédentes, **caractérisé en ce que** le premier bouillon de fermentation (2) contenant le lactate est mélangé à un sel de calcium et que le lactate d'ammonium est séparé du premier bouillon de fermentation (2) sous forme de calcium-lactate d'ammonium (5).

9. Procédé selon l'une des revendications précédentes, **caractérisé en ce que**, avant ou après la séparation du lactate d'ammonium, une partie du premier bouillon de fermentation (2) est concentrée ou séchée en une composition contenant des bactéries en retirant l'eau pour produire une préparation (4a) contenant des bactéries probiotiques.

10. Procédé selon l'une des revendications précédentes, **caractérisé en ce qu'**au moins une partie du méthane produit est oxydé et que la température du premier bouillon de fermentation est équilibrée par la chaleur qui est produite par l'oxydation.

11. Dispositif pour produire du lactate d'ammonium à l'aide d'un premier récipient de fermentation (21) qui est conçu pour accueillir une solution (1) aqueuse contenant un premier substrat pour une fermentation d'acide lactique et qui comporte un premier conduit d'arrivée (24) pour le NH₃ (13) gazeux et un conduit de prélèvement (22) pour prélever le premier bouillon de fermentation (2) contenant du lactate,
**caractérisé en ce que** le premier conduit d'arrivée (24) est relié à un conduit de décharge, destiné au NH₃ (13) et relié à un deuxième récipient de fermentation (31),
et un dispositif de strippage (33) est placé entre le deuxième récipient de fermentation (31) et le premier conduit d'arrivée (24) pour stripper le NH₃ et
le dispositif de strippage (33) comporte un conduit d'arrivée destiné à l'agent de strippage et relié à une conduite d'aération du premier récipient de fermentation (21),
le deuxième récipient de fermentation (31) étant conçu pour la transformation microbienne méthanogène de biomasse (10) contenant du protéine, de l'acide urique et/ou de l'urée en un biogaz (11) qui contient du méthane et en un deuxième bouillon de fermentation de biogaz contenant du NH₃,
et le premier récipient de fermentation (21) comprend un premier échangeur thermique qui est couplé, à l'aide d'un conduit d'arrivée et d'un conduit de prélèvement destinés à l'agent d'échange thermique, à un deuxième échangeur thermique placé sur le deuxième récipient de fermentation (31).

12. Dispositif selon la revendication 11, **caractérisé en ce qu'**un séparateur muni d'une sortie pour une phase liquide et d'une sortie pour une phase solide est placé entre le deuxième récipient de fermentation (31) et le premier conduit d'arrivée (24), le dispositif de strippage (33) étant raccordé à la sortie destinée à la phase liquide.

13. Dispositif selon l'une des revendications 11 à 12, **caractérisé en ce que** le conduit de sortie comporte un récipient tampon (35) pour recueillir le NH₃ (13) qui se présente sous forme gazeuse ou en solution aqueuse.

14. Dispositif selon l'une des revendications 11 à 13, **caractérisé en ce qu'**un dispositif de séparation (23) est relié au conduit de prélèvement (22) destiné à prélever le premier bouillon de fermentation (2) contenant du lactate, lequel dispositif de séparation est conçu pour séparer le lactate d'ammonium du premier bouillon de fermentation (2) et comporte un conduit destiné à acheminer dans le deuxième récipient de fermentation (31) la fraction du premier bouillon de fermentation (2) de laquelle est séparé le lactate d'ammonium.

15. Dispositif selon l'une des revendications 11 à 14, **caractérisé en ce que** le premier conduit d'arrivée (24) destiné au NH₃ (13) gazeux est relié à la sortie du dispositif de strippage (33) destiné au gaz contenant du NH₃.
